# EUROPEAN PATENT SPECIFICATION

(11) **EP 2 985 019 B1**
(45) Date of publication and mention of the grant of the patent: **20.10.2021**
(21) Application number: 14181206.5
(22) Date of filing: 16.08.2014
(51) Int. Cl.: A61K 9/00, A61K 47/10, A61K 31/7028, A61P 31/16

(54) **Nasal composition having anti-viral properties**
Nasale Zusammensetzung mit antiviralen Eigenschaften
Composition nasale ayant des propriétés antivirales

(43) Date of publication of application: 17.02.2016
(73) Proprietor: Church & Dwight Co., Inc., Princeton, NJ 08543-5297 (US)
(72) Inventor: Saaid, Amina, 60870 Rieux (FR)
(74) Representative: August Debouzy

(56) References cited:
- EP-A1- 1 407 776
- WO-A1-2005/103382
- WO-A1-2010/151707
- WO-A2-99/15147
- WO-A2-2006/050489
- WO-A2-2006/091722
- WO-A2-2007/091037

## Description

### FIELD OF THE INVENTION

The present invention refers to nasal compositions, having anti-viral properties.

### TECHNICAL BACKGROUND

Nasal sprays are known in the art.

The applicant is manufacturing nasal sprays currently on the market under the trademark Sterimar®. Three main grades are available, as follows. The copper formulation is dedicated to blocked nose, the manganese formulation is dedicated to allergic nose and the sulfur composition is dedicated to irritated nose.

WO2010151707 discloses alkylglycoside-containing compositions and methods for preventing loss of a parathyroid hormone (PTH) analog or octreotide via denaturation due to adherence upon contact with glass.

WO2007091037 discloses virucidal compositions and their use in preventing and/or treating a spectrum of viral species, compositions comprising water, an acid, an ammonium agent and a water-soluble zinc, copper, selenium and/or manganese compound.

It is also known to use in a nasal spray a specific grade of hyaluronic acid. FR2847818 discloses the use of a low MW hyaluronic acid, with a MW below 10⁵ Daltons. It is also indicated that the mucociliary clearance is improved with low MWs compared to high MWs.

Yet, the compositions of the prior art still requires improvements, notably for the antiviral activity and the film-forming activity.

### SUMMARY OF THE INVENTION

The invention provides a nasal composition comprising:
- a saline base;
- an active agent comprising manganese in combination with copper;
- an alkyl poly glucoside; and
- a mixture of a first hyaluronic acid having a molecular weight of 20,000 to 50,000 daltons with a second hyaluronic acid having a molecular weight of 100,000 to 300,000 daltons; according to claim 1

According to one embodiment, the composition comprises from 0.01 to 1, preferably from 0.05 to 0.5 g, more preferably from 0.1 to 0.3 g of alkyl poly glucoside per 100 ml of nasal spray composition.

According to one embodiment, the alkyl poly glucoside is selected from the list consisting of decyl glucoside, coco glucoside, lauryl glucoside, hexyl glucoside, butyl glucoside, caprylyl/capryl glucoside and mixtures thereof.

According to one embodiment, the composition is hypotonic, isotonic or hypertonic, preferably isotonic or hypertonic.

According to one embodiment, the first and second hyaluronic acids are present according to a weight ratio of 10:1 to 1:10, preferably from 5:1 to 1:5, more preferably from 3:1 to 1:3, more advantageously from 2:1 to 1:2 and especially about 1:1.

According to one embodiment, the first and second hyaluronic acids are each present in amounts from 0.01 to 1 g, preferably from 0.05 to 0.5 g, more preferably from 0.1 to 0.3 g of hyaluronic acid per 100 ml of nasal composition.

According to one embodiment, the active agent is a mixture of copper and manganese, in the form of metallic salt(s), preferably as hypertonic composition.

According to one embodiment, the composition is a spray.

The composition of the invention is notably for the treatment of pathologies of the upper airways.

The invention also provides a dispenser, preferably equipped with a pump, containing the nasal composition of the invention.

The invention also describes the alkyl poly glucoside as an anti-viral agent, preferably for the prophylactic or therapeutic treatment of rhinovirus infections.

According to one embodiment, the alkyl poly glucoside of the present invention is selected from the list consisting of decyl glucoside, coco glucoside lauryl glucoside, hexyl glucoside, butyl glucoside, caprylyl/capryl glucoside and mixtures thereof.

The compositions of the invention thus:
- encapsulates foreign particles (allergens, dust, pollution, etc.) responsible for the allergic reaction, inactivates and removes them quickly ;
- provides a protective, imperceptible, film on the nasal mucosa which protects it from further attacks, soothes and hydrates it ;
- significantly strengthens the barrier function in full respect of the mucosa.

The invention also allows for improved anti-viral efficacy.

The invention further provides advantages as will be apparent from the description.

### BRIEF DESCRIPTION OF THE DRAWINGS

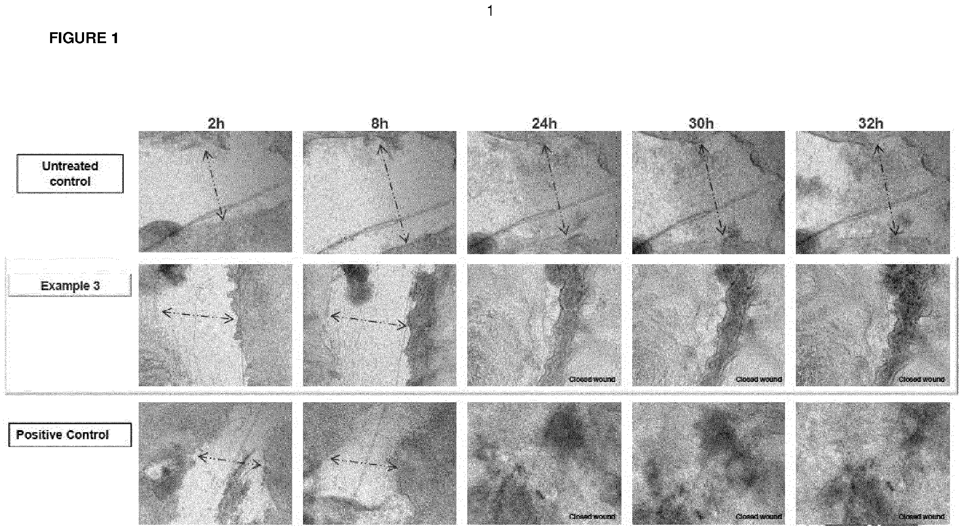

- Figure 1 is a series of microphotographs re-epithelization for a untreated control, example 3 and positive control.

### DESCRIPTION OF EMBODIMENTS OF THE INVENTION

The invention is now disclosed in more details in the following description. Unless specified otherwise, amounts and percentages are by weight. Molecular weights are expressed in Daltons (or kilodaltons).

### Saline base.

The saline base is the one typically used in nasal compositions. The composition containing the saline base can be hypotonic, isotonic or hypertonic (including slightly hypertonic), depending on the specific use that is intended for the nasal spray. The salt used in the saline base can be the typical sea salts or sodium chloride, since the saline composition typically comprises filtered sea water, but other salts can be used. For example, calcium chloride salt can be used.

Preferred compositions are isotonic (i.e containing about 32 ml of sea water without added salt, for a total volume of aqueous composition of 100 ml) or are hypertonic, notably with 45 to 80 ml sea water per 100 ml, preferably from 50 to 75 ml sea water per 100 ml of composition.

### Active agent

An active ingredient is typically used in the nasal sprays. A metallic salt is typically used in a nasal spray, such as a copper salt and/or a manganese salt. Sulfur is also used in nasal sprays, notably as thio compounds. The Applicant is already marketing nasal sprays containing said agent, where each agent is more specifically dedicated to one specific pathology or symptom. In summary, the copper formulation is dedicated to blocked nose, the manganese formulation is dedicated to allergic nose and the sulfur composition is dedicated to irritated nose.

This is already in use in the commercial Sterimar® products, marketed by the Applicant, as recalled above.

The respective amounts can be varied according to the extent of treatment, the duration, the volume sprayed into the nose, and the like. It has been found that the amounts used in the existing commercial compositions are appropriate for the respective treatments.

A drug can be present as well. Possible drugs are disclosed in WO01/93846, see page 5, lines 3-13. Further possible drugs are also disclosed in WO2006/003521, at paragraphs [0081] to [0094]. A steroid or an antihistamine can for example be present.

A formulation with copper will preferably be hypertonic.

A formulation with manganese will preferably be (slightly) hypertonic.

A formulation with sulfur will preferably be isotonic.

### Hyaluronic compound

The hyaluronic compound of the invention is a mixture of two grades of hyaluronic acid (HA). The term hyaluronic acid also encompasses salts and derivatives thereof, especially sodium hyaluronate.

The first part is the low molecular weight (low MW) hyaluronic acid disclosed in FR2847818, which is incorporated herein by reference. It can be prepared according to the general processes of fermentation, as is recalled in said document FR2847818.

The low MW hyaluronic acid has a molecular weight at most 10⁵ Da, preferably at most 5.10⁴ Da, more preferably between 20 000 and 50 000.

It is also available on the market by the supplier Soliance, under the tradename PrimalHyal 50/Renovhyal.

The second part is the medium molecular weight (medium MW) hyaluronic acid. It can be prepared according to the general biotechnological processes.

The medium MW hyaluronic acid has a molecular weight at least 10⁵ Da, preferably at most 5.10⁵ Da, more preferably between 100 000 and 300 000.

It is also available on the market by the supplier Soliance, under the tradename PrimalHyal 300/Bashyal.

The amount of each of the low and medium MW hyaluronic acid can be from 0.01 to 1 g, preferably from 0.05 to 0.5 g, more preferably from 0.1 to 0.3 g of hyaluronic acid (low MW or medium MW) per 100 ml of composition of the invention.

The weight ratio of low MW to medium MW HA is from 10:1 to 1:10, preferably from 5:1 to 1:5, more preferably from 3:1 to 1:3, more advantageously from 2:1 to 1:2 and especially about 1:1.

The specific use of two different HAs with different MWs allows an improved film-forming capacity for the composition, and provides the advantages as evidenced in the examples section.

### Alkyl poly glucoside

Alkyl poly glucoside (APG) is a known surfactant. It is a non-ionic surfactant generally of vegetal origin (synthetic origin is also possible), which can be advantageously from renewable materials. It can be selected from the list consisting of decyl glucoside, coco glucoside, lauryl glucoside, hexyl glucoside, butyl glucoside, caprylyl/capryl glucoside (the later being mixed C₈-C₁₀ chain), and mixtures thereof.

The composition comprising the APG (especially in association with the HA) has an anti-viral effect.

The alkyl poly glucoside used in the present invention can be present in amounts from 0.01 to 1, preferably from 0.05 to 0.5 g, more preferably from 0.1 to 0.3 g of alkyl poly glucoside per 100 ml of nasal composition.

### Other ingredients

Any other ingredient commonly used can be present. For example, essential oils can be incorporated if it is so wished. Surfactants, viscosity modifiers, preservatives, coloring agents and the like can also be present, albeit this is not especially preferred.

### Dispensing device.

The composition of the invention can be placed in an appropriate dispensing device for it to be dispensed, preferably as a nasal spray. Preferably a pump system is used, so as to avoid propellants. The use of specific pumps further allows to have preservative-free compositions; it is however possible to have compositions with a preservative. Metered dosing pumps are preferred, where the typical volume of metered dispensed composition is at least 100µl, typically 140µl. The volume of the housing can be for example less than 100ml, typically about 20ml.

However, any type of dispensing devices is appropriate including pressurized vessels using pressuring gas (such as nitrogen). Typical sprays dispensing devices, such as bag-on-valve.

### Therapeutical uses and pharmaceutical compositions.

The composition of the invention is useful for the treatment of a great variety of diseases of the upper airways, for example inflammatory, infectious or allergic rhinitis, acute or chronic inflammatory bronchitis (infectious, allergic or toxic), chronic obstructive pulmonary disease or cystic fibrosis, rhinopharyngitis, cold, bronchiectasia, mucoviscidose, asthma and the like.

The viruses which can be treated thanks to the invention are very diverse. They can be found for example in WO2006/003521, at paragraphs [0049] to [0060], which is incorporated herein by reference. The invention is especially applicable to the treatment of rhinovirus infection, e.g. an acute or chronic rhinovirus infection. The rhinovirus infection can be in an individual being a high-risk patient selected from the group consisting of an asthma patient, a person suffering from allergy, and a person suffering from an inflammatory disease.

In a preferred embodiment the pharmaceutical composition is adapted for topical or mucosal use and is for example available as sprays, foams, or liquid solutions such as skin lotions, gargle, solutions or nose drops, preferably as nose drops and nasal sprays.

Usually, the composition will be provided as a non-pyrogenous, sterile preparation. In case of a liquid preparation sterility may be achieved, for example, by filtration through a suitable membrane filter. Methods for the manufacture of sterile or aseptic pharmaceutical compositions are well known in the art.

A dose would typically be two pulverizations into each nostril, for example up to 4 to 10, preferably 6 times per day, and generally up to 4 to 10 consecutive days, typically up to 6 days. The typical content of a dose is about 140µl.

The invention is also directed towards compositions and methods for the prophylactic or therapeutic treatment of diseases of the upper airways, preferably a rhinovirus infection. Notably the method comprises the steps of administering, preferably by pulverization or spraying in the nose, a composition of the invention to a patient in need thereof.

### EXAMPLES

The following examples illustrate the invention without limiting it.

### Example 1 -« blocked nose » composition (anti-viral composition)

The following composition is prepared by adding to the existing Sterimar® "blocked nose" composition the following ingredients:

| Ingredient | Unit formula |
|---|---|
| APG | 0.1-0.3 g |
| HA (20-50kDa) | 0.1-0.3 g |
| HA (100-300kDa) | 0.1-0.3 g |

This composition will :
   - Decongests rapidly
   - Induce a protective effect at the tight junctions level
   - Improve the mucociliary clearance
   - Stimulate the phagocytic activity
   - Exhibit anti-bacterial potential against *Staphylococcus Aureus*
   - Inhibit the replication of HRV providing for prophylactic activity and preventing and fighting infection and surinfection

### Example 2 - « allergic nose » composition

The following composition is prepared by adding to the existing Sterimar® "allergic nose" composition the following ingredients:

| Ingredient | Unit formula |
|---|---|
| Sea water | qsp 50.00 ml |
| HA (20-50kDa) | 0.1-0.3 g |
| HA (100-300kDa) | 0.1-0.3 g |

This composition will :
   - Induce a protective effect at the tight junctions level
   - Improve the mucociliary clearance
   - Stimulate the phagocytic activity

### Example 3 - « irritated nose » composition

The following composition is prepared by adding to the existing Sterimar® "irritated nose" composition the following ingredients:

| Ingredient | Unit formula |
|---|---|
| HA (20-50kDa) | 0.1-0.3 g |
| HA (100-300kDa) | 0.1-0.3 g |

This composition will :
- Induce a protective effect at the tight junctions level
- Have an healing effect on injured epithelium
- Allow a re-epithelialization in full respect of the barrier structure without pharmacological effect.

Each of the three formulations is submitted to various tests, as reported below, evidencing the benefits of the various formulations.

### Cell integrity, cytotoxicity and irritation study.

A 3D model of the human airway epithelia is used as a sensitive and reliable model for in-vitro toxicology and pharmaco-toxicological testing. It is a human nasal epithelium which is reconstituted and is available from the company Epithelix under the tradename MucilAir™. The test provides for TEER (Trans Epithelial Electrical Resistance) measurement (known method to functionally analyze tight junction dynamics in cell culture models of barriers and permeability of epithelium), Resazurin test, Mucociliary clearance and morphology.

### Rezasurin cytotoxicity assay.

This cytotoxicity assay results establish that all formulations show no cytotoxicity and no irritation. For untreated cultures, the value is at 100%. The same value is achieved for all examples 1, 2 and 3.

### Membrane integrity test.

Membrane integrity monitoring is determined using Lactate DeHydrogenase (LDH), which is an enzyme found extensively in body tissues, normally present into the cell (Cytosol) and released during tissue damage. It is a marker of common injuries and disease; the measure of LDH reflects the state of cells: its absence in the extracellular medium equating membrane integrity.

The LDH assay results establish tissue integrity at day 1, 2, 3 and 4. This is apparent from assays comparing the formulations of examples 1, 2 and 3 with saline solution and untreated cultures. None of them exhibit any cytotoxicity as measured using LDH as a marker.

### Pro-inflammatory effect test.

The airway epithelia is a physical barrier and modulator of immune responses and its cells synthesize and release a large panel of inflammatory mediators (cytokines and chemokines) upon external pathogen/chemical challenge. Interleukin 8 (IL-8) is one of the most abundant cytokines released by the airway epithelial cells after inflammatory stimuli. It is a reliable biomarker for detecting the adverse effects of the chemicals. It is a good marker for allergic response.

The IL-8 assay results establish tissue integrity at day 1, 2, 3 and 4. This is apparent from the below table, comparing the formulations of examples 1, 2 and 3 with saline solution and untreated cultures as well as a positive control. The following table provides the values expressed in ng/ml, at days 1, 2, 3 and 4

| | IL-8 D1 | IL-8 D2 | IL-8 D3 | IL-8 D4 |
|---|---|---|---|---|
| Untreated cultures | 5.6 | 8.6 | 12.0 | 14.6 |
| Saline Solution | | 6.7 | 14.5 | 32.8 |
| Example 1 | 10.1 | 33.4 | 19.4 | 11.4 |
| Example 2 | 11.8 | 9.8 | 10.0 | 8.7 |
| Example 3 | 5.2 | 4.5 | 8.9 | 6.0 |
| Positive control | 201.1 | 268.3 | 264.8 | 278.0 |

### Film-forming efficacy of example 1 composition

The film-forming efficacy is deduced by the measurement of the TEER at T=0, then at T=72h, compared to untreated control.

An increase in the value is evidencing the film-forming capability of the tested composition. The result is given in the table graph below where the saline solution is the negative control and 100% is the arbitrary value given at T=0; results are expressed as %.

| | Saline solution | Example 1 |
|---|---|---|
| T=0h | 100 | 100 |
| T=72h | 93 | 223 |

### Mucociliary clearance of example 1 composition

The mucociliary clearance is an important primary innate defense mechanism that protects the lungs from deleterious effects of inhaled pollutants, allergens, and pathogens in the nose; mucus is moved toward the pharynx by cilia beating.

The result is given in the table below which provides the mucociliary clearance at days 1 and 4, expressed in µm/s, for the untreated cultures, the saline solution, the composition of the example and a positive control.

| | MCC D1 | MCC D4 |
|---|---|---|
| Untreated cultures | 20.42 | 20.73 |
| Saline Solution | 49.22 | 39.06 |
| Example 1 | 50.34 | 50.53 |
| Positive control | 54.50 | 48.32 |

### Phagocytosis of example 1 composition.

Phagocytosis is a cellular process of foreign particle ingestion:
- The virus, bacteria, allergens or foreign particle is engulfed
- Internalized in a vesicle into the cell (Phagosome)
- Usually the content of this vesicle is destroyed and eliminated.

The phagocytosis assay is based on latex fluorescent micro-beads (as foreign particles) after 1 h treatment with the product.

The tissue treated for 1h with the products induced an increase in fluorescence intensity expressed as RFU (relative fluorescence unit) compared to the control cells (with saline solution) corresponding to an increase in phagocytic activity for example 1 composition. The result is given in the table below.

| | Control | Example 1 |
|---|---|---|
| RFU | 13405 | 44016 |
| Fold increase | 1 | 3.3 |

Allergens and viruses would be more rapidly eliminated, reducing the risks of negative effect of allergens and viruses.

### Anti-bacterial activity of example 1 composition.

Monitoring of cultivable Staphylococcus aureus densities after tested strains seeding in the formulations allows determination of the bactericidal effect. The table below evidences that the composition exhibits a bactericidal effect from 1 hour, by measuring the densities expressed in UFC/ml for times of 1, 3 and 24 hours.

| | 0 | 1 | 3 | 24 |
|---|---|---|---|---|
| Growth control | 3.2 10⁶ | 4.4 10⁶ | 2.2 10⁷ | 5.10 10⁹ |
| Example 1 | 3.2 10⁶ | 1 | 1 | 1 |

### Anti-viral effect of example 1 composition.

The example 1 composition is tested according to the test of evaluation of the potential activity against HRV-A16 (a rhinovirus responsible for colds) on fully differentiated human airway epithelial cells culture. The compositions are first applied before infection for one hour and the replication is carried out for 4 hours. 3 rinsing steps with the formulations are carried out. The remaining is collected and the RNA is dosed after cell lysis. The same collecting step is carried out 24 hours after and the same dosing step is carried out. The percentage expressed the changes in viral RNA, resulting in the % of inhibition of HRV-A16 replication, leading to the anti-viral efficacy. The tested composition is tested against rupintrivir (a known antiviral compound, providing an almost complete response). The results are the following:

| Product | Inhibition (%) |
|---|---|
| Rupintrivir | 99.62 |
| Example 1 formulation | 99.8 |

### Film-forming efficacy of example 2 composition

The film-forming efficacy is deduced by the measurement of the TEER at T=0, then at T=72h, compared to untreated control.

An increase in the value is evidencing the film-forming capability of the tested composition. The result is given in the table graph below where the saline solution is the negative control and 100% is the arbitrary value given at T=0; results are expressed as %.

| | Saline solution | Example 2 |
|---|---|---|
| T=0h | 100 | 100 |
| T=72h | 93 | 127 |

### Mucociliary clearance of example 2 composition

The mucociliary clearance is an important primary innate defense mechanism that protects the lungs from deleterious effects of inhaled pollutants, allergens, and pathogens in the nose, mucus is moved toward the pharynx by cilia beating.

The result is given in the table below which provides the mucociliary clearance at days 1 and 4, expressed in µm/s, for the untreated cultures, the saline solution, the composition of the example and a positive control.

| | MCC D1 | MCC D4 |
|---|---|---|
| Untreated cultures | 20.42 | 20.73 |
| Saline Solution | 49.22 | 39.06 |
| Example 2 | 34.85 | 61.29 |
| Positive control | 54.50 | 48.32 |

### Phagocytosis of example 2 composition.

Phagocytosis is a cellular process of foreign particle ingestion:
- The virus, bacteria, allergens or foreign particle is engulfed
- Internalized in a vesicle into the cell (Phagosome)
- Usually the content of this vesicle is destroyed and eliminated.

The phagocytosis assay is based on latex fluorescent micro-beads (as foreign particles) after 1 h treatment with the product.

The tissue treated for 1h with the products induced an increase in fluorescence intensity expressed as RFU (relative fluorescence unit) compared to the control cells (with saline solution) corresponding to an increase in phagocytic activity for example 2 composition. The result is given in the table below.

| | Control | Example 2 |
|---|---|---|
| RFU | 13405 | 16271 |
| Fold increase | 1 | 1.2 |

Allergens would be more rapidly eliminated, reducing the risks of negative effect of allergens.

### Film-forming efficacy of example 3 composition

The film-forming efficacy is deduced by the measurement of the TEER at T=0, then at T=72h, compared to untreated control.

An increase in the value is evidencing the film-forming capability of the tested composition. The result is given in the table graph below where the saline solution is the negative control and 100% is the arbitrary value given at T=0; results are expressed as %.

| | Saline solution | Example 3 |
|---|---|---|
| T=0h | 100 | 100 |
| T=72h | 113 | 146 |

### Re-epithelialization activity of example 3 composition.

The example 3 composition induced a strong front of proliferation and migration at the edge of the gap (visible difference between 8h and 24h). At 24h the wound is completely closed. The healing activity of the example 3 composition is similar to a positive control.

This is visible from the comparison of the microphotographs of figure 1.

The increase in occludin suggests its involvement in stabilizing the tight junction maintaining the barrier integrity helping the re-epithelialization process. The increase in integrin β1 facilitates the migration of epithelial cells into the wounded area by organizing the cell cytoscheleton. This is apparent from the table below, including a positive control and evidencing the efficacy of the composition; the saline solution (negative control) being arbitrarily set at the value of 1 and the other value being relative. There is a wound healing due to a mechanical effect (substantially without pharmacological effect), since the composition stimulates both occludin and integrin β1, resulting in cells migration rather than proliferation as would do a drug.

| Sample | Occludin | Integrin β1 |
|---|---|---|
| Saline solution | 1 | 1 |
| Injured epithelia | 1.1745 | 1.37 |
| Example 3 | 1.933 | 2.105 |
| Positive control | 0.912 | 1.446 |

## Claims

1. Nasal composition comprising:
- a saline base ;
- an active agent comprising manganese in combination with copper;
- an alkyl poly glucoside; and
- a mixture of a first hyaluronic acid having a molecular weight of 20,000 to 50,000 daltons with a second hyaluronic acid having a molecular weight of 100,000 to 300,000 daltons.

2. Nasal composition of claim 1, comprising from 0.01 to 1, preferably from 0.05 to 0.5 g, more preferably from 0.1 to 0.3 g of alkyl poly glucoside per 100 ml of nasal spray composition.

3. Nasal composition of claim 1, wherein the alkyl poly glucoside is selected from the list consisting of decyl glucoside, coco glucoside, lauryl glucoside, hexyl glucoside, butyl glucoside, caprylyl/capryl glucoside and mixtures thereof.

4. Nasal composition of claim 1, wherein the composition is hypotonic, isotonic or hypertonic, preferably isotonic or hypertonic.

5. Nasal composition of claim 1, wherein the first and second hyaluronic acids are present according to a weight ratio of 10:1 to 1:10, preferably from 5:1 to 1:5, more preferably from 3:1 to 1:3, more advantageously from 2:1 to 1:2 and especially about 1:1.

6. Nasal composition of claim 1, wherein the first and second hyaluronic acids are each present in amounts from 0.01 to 1 g, preferably from 0.05 to 0.5 g, more preferably from 0.1 to 0.3 g of hyaluronic acid per 100 ml of nasal composition.

7. Nasal composition of claim 1, wherein the active agent is a mixture of copper and manganese in the form of metallic salt(s), preferably as hypertonic composition.

8. Nasal composition of any one of claims 1 to 7, which is a spray.

9. Nasal composition of any one of claims 1 to 8, for use in the treatment of pathologies of the upper airways.

10. A dispenser, preferably equipped with a pump, containing the nasal composition of any one of the claims 1 to 9.

## Patentansprüche

1. Nasalzusammensetzung, umfassend:
- eine salzhaltige Basis;
- einen Wirkstoff, der Mangan in Kombination mit Kupfer umfasst;
- ein Alkylpolyglucosid; und
- ein Gemisch aus einer ersten Hyaluronsäure mit einem Molekulargewicht von 20.000 bis 50.000 Dalton mit einer zweiten Hyaluronsäure mit einem Molekulargewicht von 100.000 bis 300.000 Dalton.

2. Nasalzusammensetzung nach Anspruch, umfassend 0,01 bis 1 g, vorzugsweise 0,05 bis 0,5 g und insbesondere 0,1 bis 0,3 g Alkylpolyglucosid pro 100 ml Nasensprayzusammensetzung.

3. Nasalzusammensetzung nach Anspruch, wobei das Alkylpolyglucosid aus der Liste bestehend aus Decylglucosid, Kokosglucosid, Laurylglucosid, Hexylglucosid, Butylglucosid, Caprylyl-/Caprylglucosid und Mischungen davon ausgewählt ist.

4. Nasalzusammensetzung nach Anspruch 1, wobei die Zusammensetzung hypotonisch, isotonisch oder hypertonisch, vorzugsweise isotonisch oder hypertonisch ist.

5. Nasalzusammensetzung nach Anspruch 1, wobei die erste und die zweite Hyaluronsäure gemäß einem Gewichtsverhältnis von 10:1 bis 1:10, vorzugsweise 5:1 bis 1:5, insbesondere 3:1 bis 1:3, vorteilhafterweise 2:1 bis 1:2 und speziell etwa 1:1 vorhanden sind.

6. Nasalzusammensetzung nach Anspruch 1, wobei die erste und die zweite Hyaluronsäure jeweils in Mengen von 0,01 bis 1 g, vorzugsweise 0,05 bis 0,5 g und insbesondere 0,1 bis 0,3 g Hyaluronsäure pro 100 ml Nasalzusammensetzung vorhanden sind.

7. Nasalzusammensetzung nach Anspruch 1, wobei der Wirkstoff ein Gemisch aus Kupfer und Mangan in Form von Metallsalz(en) vorzugsweise als hypertonische Zusammensetzung ist.

8. Nasalzusammensetzung nach einem der Ansprüche 1 bis 7, wobei es sich um ein Spray handelt.

9. Nasalzusammensetzung nach einem der Ansprüche 1 bis 8 zur Verwendung bei der Behandlung von Pathologien der oberen Atemwege.

10. Spender, der vorzugsweise mit einer Pumpe ausgestattet ist und die Nasalzusammensetzung nach einem der Ansprüche 1 bis 9 enthält.

## Revendications

1. Composition nasale comprenant :
- une base saline ;
- un principe actif comprenant du manganèse en combinaison avec du cuivre ;
- un alkylpolyglucoside ; et
- un mélange d'un premier acide hyaluronique ayant un poids moléculaire de 20 000 à 50 000 daltons avec un deuxième acide hyaluronique ayant un poids moléculaire de 100 000 à 300 000 daltons.

2. Composition nasale selon la revendication 1, comprenant de 0,01 à 1, de préférence de 0,05 à 0,5 g, mieux encore de 0,1 à 0,3 g d'alkylpolyglucoside pour 100 ml de composition de spray nasal.

3. Composition nasale selon la revendication 1, dans laquelle l'alkylpolyglucoside est choisi dans la liste consistant en le décylglucoside, le (coprahyl)glucoside, le laurylglucoside, l'hexylglucoside, le butylglucoside, le caprylyl/caprylglucoside et leurs mélanges.

4. Composition nasale selon la revendication 1, laquelle composition est hypotonique, isotonique ou hypertonique, de préférence isotonique ou hypertonique.

5. Composition nasale selon la revendication 1, dans laquelle les premier et deuxième acides hyaluroniques sont présents en un rapport en poids de 10/1 à 1/10, de préférence de 5/1 à 1/5, mieux encore de 3/1 à 1/3, plus avantageusement de 2/1 à 1/2, et en particulier d'environ 1/1.

6. Composition nasale selon la revendication 1, dans laquelle les premier et deuxième acides hyaluroniques sont présents chacun en des quantités de 0,01 à 1 g, de préférence de 0,05 à 0,5 g, mieux encore de 0,1 à 0,3 g d'acide hyaluronique pour 100 ml de composition nasale.

7. Composition nasale selon la revendication 1, dans laquelle le principe actif est un mélange de cuivre et de manganèse sous la forme d'un ou plusieurs sels métalliques, de préférence sous la forme d'une composition hypertonique.

8. Composition nasale selon l'une quelconque des revendications 1 à 7, qui est un spray.

9. Composition nasale selon l'une quelconque des revendications 1 à 8, pour une utilisation dans le traitement de pathologies des voies aériennes supérieures.

10. Distributeur, de préférence équipé d'une pompe, contenant la composition nasale de l'une quelconque des revendications 1 à 9.
